Europäisches Patentamt

European Patent Office

Office européen des brevets

(11) Publication number: **0 153 647**
**A2**

# (12) EUROPEAN PATENT APPLICATION

(21) Application number: **85101446.4**

(51) Int. Cl.⁴: **A 61 B 17/39**

(22) Date of filing: **11.02.85**

(30) Priority: **02.03.84 US 585565**

(43) Date of publication of application:
**04.09.85 Bulletin 85/36**

(84) Designated Contracting States:
**AT BE CH DE FR GB IT LI LU NL SE**

(71) Applicant: **DART INDUSTRIES INC.**
**2211 Sanders Road**
**Northbrook, IL 60062(US)**

(72) Inventor: **Pool, L. Franklin**
**6505 N.W. Cherry Street**
**Vancouver Washington 98663(US)**

(74) Representative: **Howden, Christopher Andrew et al,**
**FORRESTER & BOEHMERT Widenmayerstrasse 4/I**
**D-8000 München 22(DE)**

(54) An electrosurgical pencil.

(57) The present invention is comprised of an electrosurgical pencil of the type where current is provided from an electrosurgical generator to the blade of the pencil through a hot lead whenever a switching circuit is activated by interconnecting the hot lead with a switching lead which also runs from the pencil to the generator. The pencil isolates the hot lead from the blade at all times when the switching circuit is not also activated by that pencil. Accordingly, when the generator is caused to transmit current by the activation of one pencil, the current will not reach the blade of other pencils connected to the same generator.

FIG. 3

EP 0 153 647 A2

"An Electrosurgical Pencil"

This invention relates to an electrosurgical pencil and in particular to such a pencil where a hot lead, through which current is transmitted to the blade, is isolated from the blade until the pencil has been activated.

It is well known in the prior art to use high frequency current to perform surgical functions. Typically, in surgical equipment using high frequency current, an electrosurgical generator is used to generate current in a desired wave form upon demand and this current is carried to an electrosurgical pencil having a small blade. The current is transmitted through the blade to the patient and back to the generator through a ground plate attached to the patient. Since the blade is quite small, relative to the ground plate, the energy being transferred to the patient through the blade is concentrated on a small area and the resulting high frequency oscillations in that small area cause tissue destruction. Typically, such devices have two modes of operation, cutting and coagulation, which operations require current having different wave forms.

In such typical devices the current is initiated and interrupted by means of a switching circuit in the generator which is controlled either by a switch in the pencil or by a floor switch. In the former case, a switching lead runs from the generator to the pencil, with the switching circuit in the generator being activated to provide current to the hot lead when the switching lead and hot lead are electrically interconnected at the pencil. Thus, there are typically three leads to pencil; a hot lead which runs from the generator to the blade and two switching leads which run from the generator to the pencil. A three-position switch, which in one position connects one of the switching leads to the hot head, thereby providing cutting current to the blade, in a second position connects the other switching lead to the hot lead, thereby providing coagulating current to the blade, and in a third position electrically separates all of the leads from one another thereby providing no current to the blade, is located in the pencil.

The difficulty with such units lies in the fact that in actual operating conditions, it is typical for serveral surgeons simultaneously to use pencils which are connected to a common generator. Since the generator provides current, through the hot lead of the pencils, upon activation of the switching circuit, when one pencil is activated current is inevitably supplied to all other pencils connected to the generators. Thus, when one of the pencils is in a position where the blade comes in contact with the patient, and another pencil is activated, either intentionally or by accident, the first pencil will cause a burn to occur in the patient. While attempts have been made to design the switching circuit in the generator to provide safeguards which prevent current from accidentally flowing to the wrong pencil, no safeguards have heretofore been provided in the pencil itself to prevent accidents from occurring.

Accordingly, in a first aspect, the invention

0153647

provides an electrosurgical pencil, comprising a blade adapted to receive current from an electrosurgical current generator, lead means for transmitting said current from said generator to said pencil; and means for isolating said lead means from said blade.

Preferably, the pencil further comprises switch means associated with said pencil for causing said generator to transmit current to said lead means, said switch means also comprising said means for isolating the lead means from said blade.

According to a second aspect, the invention provides an electrosurgical pencil comprising a blade which is adapted to receive current through a hot lead from an electrosurgical current generator upon electrical interconnection of a switching lead extending between the generator and the hot lead, first switch means for making and breaking contact between said switching lead and said hot lead; second switch means for making and breaking contact between said hot lead and said blade; and means for preventing said first switch means from operating to connect said hot lead and said switching lead unless contact is also made in said second switch means.

Desirably, a plurality of such first switch means are provided each having a different switching lead connectable with a common hot lead. Said first and second switch means are normally open.

Preferably the first switch means comprises a deformable, electrically conductive dome having activation means for positioning said dome in either a relaxed position, in which said first switch means is open, or a deflected position, in which said first switch means is closed.

Conveniently, said second switch means comprises a deformable conductive element which is interposed between said dome and said activation means, such that the deformable conductive element is caused to make contact with said dome when said dome is moved into its deflected position by said activating means, but does not make contact with said dome when the dome is in its relaxed position, said conductive element being electrically connected to said blade.

Advantageously, there are two such first switch means and two such second switch means, and said activation means comprises a rocker plate having a pair of prongs depending therefrom, one of said prongs causing deflection of the dome of one of said first switch means when said rocker plate is rocked in one direction, and the other of said prongs causing deflection of the dome of the other first switch means when the rocker plate is rocked in the other direction.

In a preferred embodiment, an electrosurgical pencil embodying the invention is provided with a switch having a case with two cylindrical cavities and two slots which extend along its length and through the cavities. Located in one of the slots and extending through both of the cavities is a hot lead which receives current from an electrosurgical generator to which it is connected. Two switching leads, which also are connected to the generator, are located in spaced apart positions in the other slot. The switching leads are electrically isolated from one another and one extends through each of the cavities. The cavities have larger diameter counterbores in their upper portions with the bottoms of the counterbores being located above the bottoms of the cavities by a distance which is greater than the diameter of the leads.

Located in the cavities are deformable conductive domes having diameters approximately equal to the diameter of the counterbores. Thus, the domes are located in the counterbores with their peripheries above the leads, and therefore not in contact with them. However, when the domes are deflected their centre portions do make contact with the leads. Accordingly, by deflecting one of the domes the switching lead associated with that cavity is brought into contact with the hot lead which activates the switching circuit in the generator and causes it to transmit current through the hot lead.

Mounted on the top of the case is a thin flexible element which is electrically conductive on the side facing the domes and electrically non-conductive on the other side. Non-conductive sleeves fit in the cavity between the domes and the flexible element to keep them separated from one another. The flexible element is electrically connected to the blade of the electrosurgical pencil. Thus when the flexible element is deflected into the cavity sufficiently to deflect the dome into contact with the leads, the hot lead and the blade also become electrically interconnected. Due to the elasticity of the dome and the flexible element when the latter is released both resume their relaxed positions and electrical contact is broken both between the hot lead and the switching lead, and between the hot lead and the blade. Since the only way that current from the generator can reach the blade is upon deflection of the flexible element in the pencil carrying that blade, when the generator is caused to generate current by activation of one of several pencils, the current can not accidentally flow through to the blade of any of the other pencils.

A rocker switch located on the electrosurgical pencil has prongs which deflect the flexible element into contact with one of the domes when it is rocked in either direction. Thus, cutting current is provided when the rocker plate is rocked in one direction, and coagulating current is provided when the switch is rocked in the other direction. No current is provided when the switch is released.

In order that the invention may be more readily understood, and so that further features thereof may be appreciated, an embodiment of an electrosurgical pencil of the invention will now be described, by way of example, with reference to the accompanying drawings, in which:

FIGURE 1 is a schematic view showing the electrical circuitry of a typical prior art electrosurgical pencil;

FIGURE 2 is a schematic view showing the electrical circuitry of a pencil of the present invention.

FIGURE 3 is a side elevational view, partly broken away, showing a pencil of the present invention;

FIGURE 4 is a plan view, partially broken away to show hidden detail, of a switch which is a component of the pencil of Figure 3;

FIGURE 5 is a sectional view, taken along the line 5-5 of Figure 4, showing the switch in an open position.

FIGURE 6 is a cross-sectional view, similar to Figure 5, showing the switch in a closed position.

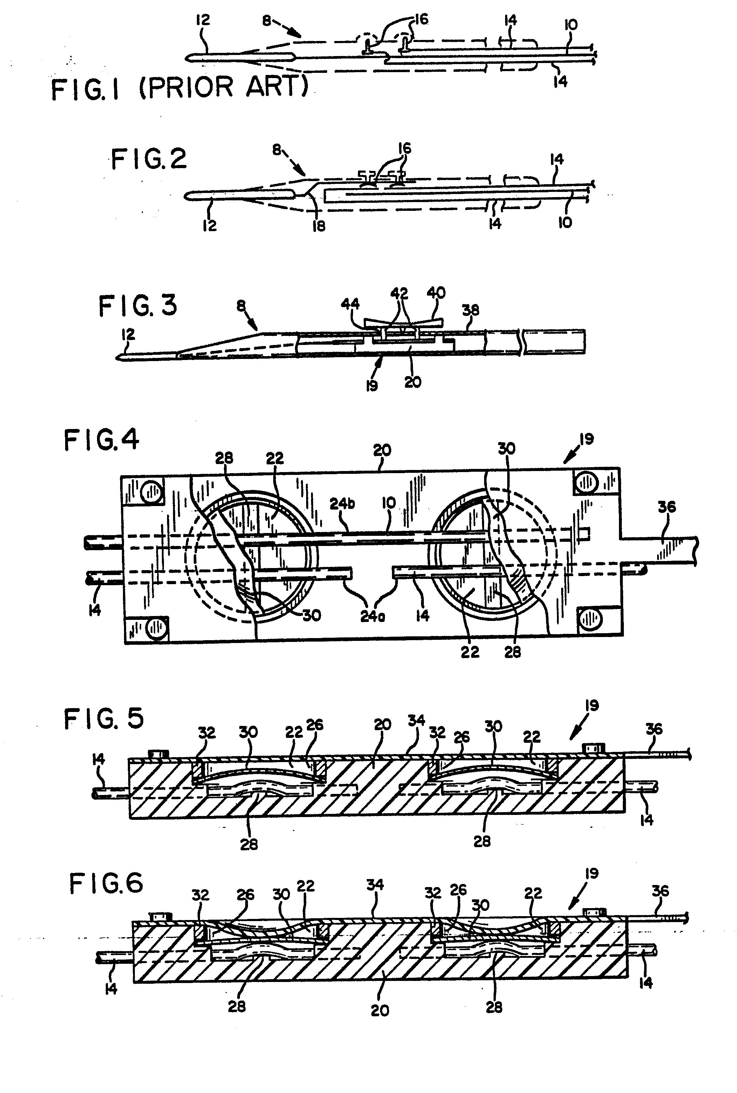

Figure 1, illustrates schematically a typical prior art electrosurgical pencil 8 having a hot lead 10 which runs uninterrupted from a generator (not shown) to a blade 12 of the pencil 8. Current flows through the hot lead 10 to the blade 12 whenever the generator is actuated. This occurs, among other times, when a switching circuit in the generator is engaged by electrically connecting one of a pair of switching lines 14 to the hot lead by depressing an appropriate switch 16.

On the other hand, in an electrosurgical pencil of the present invention, as shown schematically in Figure 2, the hot lead 10 does not extend uninterrupted from the generator (not shown) to the blade 12, and the blade 12 is normally isolated electrically from the hot lead 10. Connection is made between the hot lead 10 and the blade 12 _via_ a connecting lead 18, that extends from the blade 12 and is selectively connectable with the lead 10 by operation of a switching unit 19. Thus, the lead 18 can only be connected to the hot lead 10 when the switch unit 19 is activated to complete the switching circuit.

In a preferred embodiment of the invention, illustrated in Figures 3 to 6, the switch unit 19 comprises a case 20, shown in detail in Figures 4, 5 and 6, which carries the operative elements of the switch unit. Two cylindrical cavities 22 are located in the case 20 and two slots 24_a_ and 24_b_ extend along the length of the case to pass through the cavities 22, with the bottoms of the slots 24_a_ and _b_ being of the same depth as the bottoms of the cavities 22. In the embodiment illustrated neither of the slots 24 extend completely across the case 20. One of the slots 24_a_ is interrupted for a short distance intermediate the cavities 22, and the other slot 24_b_ terminates short of one end of the case. Neither of these interruptions of the slots is necessary, but, as will be seen later, they facilitate assembly of the switch.

The slot 24b, which extends uninterrupted from one end of the case through both of the cavities 22 receives the hot lead 10. A first switching lead 14 is located in the slots 24a and a second switching lead 14 is located in the slot 24b. Thus the hot lead 10 extends through both cavities and out of one end of the case 20 whilst each of the pair of switching leads 14 extends through only one of the cavities 22 and exits the case 20 through that end of the case in which that cavity through which the respective lead passes is located.

A counterbore 26 having a diameter slightly larger than the diameter of the cavity 22 is formed in each of the cavities 22. Each counterbore 26 terminates at a distance above the bottom of the cavity that is slightly greater than the diameter of the leads 10 and 14. Ridges are formed to extend diametrically across the bottoms of the cavities to a height which is slightly greater than the difference between the diameter of the leads, 10 and 14, and the height of the counterbores 26. Thus, the tops of the leads 10, 14 are located below the bottoms of the counterbores where they enter the cavities but are curved within the cavities to be above the bottoms of the counterbores where the leads cross the ridges 28.

An electrically conductive deformable dome 30, of the type commonly used in printed circuit board dome switches, which has a diameter substantially equal to the diameter of the counterbore 26 is located within each of the cavities 22. Due to the relative heights of the counterbores and the leads, the dome is not in contact with the leads when it is in its relaxed position, as shown in Figure 5, but, due to the ridge 28, the domes does make contact with the leads when it is deflected, as shown in Figure 6. Thus, when either of the domes 30 is deflected the switching lead entering the cavity carrying that dome is connected with the hot lead, thereby causing the generator to provide current through the hot lead. The frequency of the generated current depends upon which of the switching leads 14 is connected to the hot lead 10 and, thus, upon which of the domes 30 is deflected. A non-conductive cylindrical sleeve 32 fits above each of the domes 30, to retain this at the bottom of its respective counterbore 26.

Attached to the top of the case 20 is a thin flexible element 34 which is electrically conductive on its side facing the case and electrically non-conductive on its other side. A thin tab 36 on the element 34 extends outwardly from the case, on the end which is opposite the end which receives the hot lead 10. This tab 36 is electrically connected to the blade 12 through the lead 18. Thus the hot lead remains electrically isolated from the blade until the flexible element 34 is deflected into contact with one of the domes 30 and that dome is deflected into contact with the hot lead 10. When this occurs, as shown in Figure 6, current flows through the hot lead 10, the dome 30, the element 34 and the lead 18 to the blade 12. The electrical circuit, which extends through the patient, is completed by means of a ground connection (not shown) between the patient and the generator.

As shown in Figure 3, the case 20 is installed into an electrosurgical pencil 38 with a suitable means for activating the switch attached to the outside of the case to complete the assembly. Preferably, the activation means comprises a rocker plate 40 which has a pair of prongs 42 extending from it, one prong being located above each of the domes 30. The rocker plate 40 is pivotably attached to the pencil 38 with the prongs 42 extending through openings 44 in the pencil in a manner such that when the rocker plate is centred, neither dome is deflected and when the plate is rocked towards either end the prong on that end causes the element 34 to be deflected into contact with one of the domes 30 and the dome to be deflected into contact with the leads 10 and 14. When the rocker plate 40 is released, the resilience of the dome cause the rocker plate to be recentred allowing the element 34 and dome 30 to return to their relaxed positions. Since the element 34 is separated from the domes by the non-conductive sleeves 32, the domes remain at the bottom of the counterbores 26 and the element 34 remains separated unless deflected by contact of a prong 44 of the rocker plate 40.

The features disclosed in the foregoing description, in the following claims and/or in the accompanying drawings may, both separately and in any combination thereof, be material for realising the invention in diverse forms thereof.

CLAIMS

1. An electrosurgical pencil, comprising a blade adapted to receive current from an electrosurgical current generator, lead means for transmitting said current from said generator to said pencil; and means for isolating said lead means from said blade.

2. A pencil according to Claim 1, further comprising switch means associated with said pencil for causing said generator to transmit current to said lead means, said switch means also comprising said means for isolating the lead means from said blade.

3. An electrosurgical pencil comprising a blade which is adapted to receive current through a hot lead from an electrosurgical current generator upon electrical interconnection of a switching lead extending between the generator and the hot lead, first switch means for making and breaking contact between said switching lead and said hot lead; second switch means for making and breaking contact between said hot lead and said blade; and means for preventing said first switch means from operating to connect said hot lead and said switching lead unless contact is also made in said second switch means.

4. A pencil according to Claim 3, further comprising a plurality of such first switch means each having a different switching lead connectable with a common hot lead.

5. A pencil of Claim 3 or 4, wherein said first and second switch means are normally open.

6. A pencil according to Claim 3, 4 or 5, wherein said first switch means comprises a deformable,

0153647

electrically conductive dome having activation means for positioning said dome in either a relaxed position, in which said first switch means is open, or a deflected position, in which said first switch means is closed.

7. A pencil according to Claim 6, wherein said second switch means comprises a deformable conductive element which is interposed between said dome and said activation means, such that the deformable conductive element is caused to make contact with said dome when said dome is moved into its deflected position by said activating means, but does not make contact with said dome when the dome is in its relaxed position, said conductive element being electrically connected to said blade.

8. A pencil according to Claim 7, wherein there are two of said first switch means and two of said second switch means and said activation means comprises a rocker plate having a pair of prongs depending therefrom, one of said prongs causing deflection of the dome of one of said first switch means when said rocker plate is rocked in one direction, and the other of said prongs causing deflection of the dome of the other first switch means when the rocker plate is rocked in the other direction.

0153647

FIG.1 (PRIOR ART)

FIG.2

FIG.3

FIG.4

FIG.5

FIG.6